Europäisches Patentamt

European Patent Office

Office européen des brevets

(19)

(11) Publication number: **0 183 424**

A1

(12) # EUROPEAN PATENT APPLICATION

(21) Application number: **85308202.2**

(22) Date of filing: **11.11.85**

(51) Int. Cl.⁴: **A 61 K 47/00**
A 61 K 31/35, A 23 K 1/17
A 23 K 1/00, A 61 K 9/00

(30) Priority: **16.11.84 GB 8428952**

(43) Date of publication of application:
**04.06.86 Bulletin 86/23**

(84) Designated Contracting States:
**BE CH DE FR GB IT LI LU NL SE**

(71) Applicant: **BEECHAM GROUP PLC**
**Beecham House Great West Road**
**Brentford Middlesex TW8 9BD(GB)**

(72) Inventor: **Beal, Merle**
**12 Darenth Way**
**Horley Surrey RH6 8JZ(GB)**

(74) Representative: **Lockwood, Barbara Ann et al,**
**Beecham Pharmaceuticals Biosciences Research Centre**
**Great Burgh Yew Tree Bottom Road**
**Epsom Surrey KT18 5XQ(GB)**

(54) Veterinarily acceptable compositions of mupirocin.

(57) A veterinary composition comprising granules of mupirocin or a veterinarily acceptable salt thereof and a binder.

Preferred binders are $C_{1-6}$ alkyl ether derivatives of cellulose. These compositions can be added to animal feed for use as a growth promoter.

EP 0 183 424 A1

0183424

B1732

Novel Compositions

The present invention relates to veterinarily acceptable compositions, in particular to veterinarily acceptable compositions of Mupirocin (formerly known as Pseudomonic acid) or veterinarily acceptable salts thereof.

Mupirocin or pseudomonic acid, together with salts and esters thereof are described in U.K. Patent No. 1395907.

Mupirocin and veterinarily acceptable salts thereof are known also to be of use as growth promoters in animals in particular pigs, as described in UK patent No. 2097670. However these compounds are often found to have low stability when incorporated directly into animal feed.

We have now discovered a composition of mupirocin or veterinarily acceptable salts thereof which may be incorporated into feed with little loss of potency.

Accordingly, the present invention provides a veterinary composition comprising granules of mupirocin or a veterinarily acceptable salt thereof and a binder.

Suitably, the salt of mupirocin is a metal salt for example an alkali metal an alkaline earth metal or a silver salt.

A preferred salt is an alkaline earth metal salt.

A particularly preferred salt is a calcium salt. Suitably the calcium salt is present as a crystalline dihydrate.

Suitable binders include optionally substituted $C_{1-6}$ alkyl ether derivatives of cellulose, acacia, tragacanth, gelatin, starch or modified starches, alginic acid and salts thereof and polyvinylpyrollidone.

Preferred binders are optionally substituted $C_{1-6}$ alkyl ether derivatives of cellulose.

The cellulose derivative may include one or more optionally substituted $C_{1-6}$ alkyl group(s) which may be the same or different.

Suitably optional substituents on the $C_{1-6}$ alkyl group include hydroxy and carboxy.

Examples of suitable optionally substituted $C_{1-6}$ alkyl ether derivatives of cellulose are ethyl cellulose, hydroxypropyl methyl cellulose, methyl cellulose, sodium carboxymethylcellulose and hydroxypropyl methyl cellulose, preferably ethyl cellulose and hydroxypropyl methyl cellulose.

Suitably the granules comprise mupirocin or a veterinarily acceptable salt thereof substantially coated with the binder.

The composition may also comprise a diluent such as microcrystalline cellulose lactose, sucrose, dicalcium phosphate, starch, soya meal, calcium carbonate or whey powder.

Preferably the diluent is microcrystalline cellulose or lactose, most preferably lactose.

When a diluent is present, the granules suitably comprise matrix of mupirocin or a veterinarily acceptable salt thereof and diluent, substantially coated with a of binder.

Suitably, the mupirocin or veterinarily acceptable salt thereof(as free acid), is present in the composition in the range of from 1% to 98% w/w of the composition, more suitably 10% to 80% w/w and preferably 50%w/w.

Suitably, the binder is present in the composition in the range of from 2% to 20% w/w of the composition, preferably 5% to 10% w/w.

Suitably the diluent may be present in the range of from 0% to 97% w/w of the formulation.

When the diluent is lactose it is preferably present in the range of from 30% to 60% w/w of the formulation.

The compositions of the invention may be prepared by admixing the constitutents, preferably in powder form, with a suitable solvent, for example an organic solvent such as n-hexane or isopropanol, or water and then removing the solvent.

The process is preferably carried out at ambient or slightly elevated temperatures for example 20-60°C.

When mupirocin calcium preferably as the crystalline dihydrate, is present in the formulation a preferred temperature range is from 40°C to 60°C.

Preferably the solvent is removed in-vacuo.

The compositions of the invention may be administered to animals in any convenient veterinarily acceptable form. Suitably the formulations may be admixed with feed which can be administered in pellet form. Compositions comprising Mupirocin or a veterinarily acceptable salt thereof are preferably admixed with feed. Suitably the amount of granules is such that the pseudomonic acid content of the feed is from 5-100ppm preferably 20 ppm.

The following Examples illustrate the invention.

## Example 1

|  | % w/w |
|---|---|
| Mupirocin calcium dihydrate (equivalent to free acid) | 50 |
| Ethyl Cellulose (grade N10) | 5 |
| Lactose | to 100 |

Mupirocin calcium pseudomonate was hammer milled through an 0.04 inch screen. The milled salt was then placed in a vacuum-mixer which had a water jacket for heating the mixer contents during processing. The other dry powdered ingredients were then added. During mixing, the powders were heated to 50°C. Isopropanol was added and the mixing continued for about 10 minutes. The mass was then placed under vacuum, and mixing continued, re-heating if required, until dry granules were obtained.

## Example 2

|  | % w/w |
|---|---|
| Mupirocin calcium dihydrate | 94.7 |
| Ethyl cellulose (grade N10) | 5.3 |

The above constituents were processed as in Example 1 until dry granules were obtained.

Example 3

|  | % w/w |
|---|---|
| Mupirocin | 10.7 |
| Ethyl cellulose (grade T10) | 8.9 |
| Microcrystalline cellulose | 80.4 |

The mupirocin and microcrystalline cellulose were mixed together dry, then a 14% w/w solution of ethyl cellulose in cyclohexane was added until a wet granular mass was obtained on stirring. The mass was passed through a 20 mesh sieve and then dried at room temperature to provide dry granules.

Stability Data

1.  Mupirocin Calcium Dihydrate

The following data shows the results obtained from storage tests conducted at 20°C.

The test samples were as follows:

7029/148 = Formulation of Example 1 in meal

7029/155 = Formulation of Example 2 in meal

7029/140 = Mupirocin calcium dihydrate in meal

| Time in weeks | % of Initial Assay | | |
|---|---|---|---|
| | 7029/148 | 7029/155 | 7029/140 |
| 2 | – | – | 95.6 |
| 4 | 97.3 | 99.7 | 89.8 |
| 6 | >100 | 96.9 | 85.8 |
| 8 | 96.6 | 94.5 | 86.1 |
| 10 | – | 91.9 | 83.0 |
| 13 | 97.1 | 90.1 | 81.4 |

2.  Mupirocin

The following data shows the results obtained from storage tests conducted at 20°C. The test samples were as follows:

7029/14B = Formulation of Example 3 in feed

7029/45A = Mupirocin in feed

| Time in weeks | % of Initial Assay | |
|---|---|---|
| | 7029/14B | 7029/45A |
| 2 | 90.0 | 95.3 |
| 4 | 92.7 | 86.5 |
| 6 | 93.6 | 75.5 |
| 8 | 90.4 | 67.7 |
| 10 | - | 61.4 |
| 12 | 90.1 | 53.6 |
| 16 | - | 43.7 |

- 1 -

CLAIMS                                            C

1.    A veterinary composition comprising granules of mupirocin or a veterinarily acceptable salt thereof and a binder.

2.    A composition according to claim 1 wherein the binder is an optionally substituted $C_{1-6}$ alkyl ether derivative of cellulose, acacia, tragacanth, gelatin, starch, modified starch, alginic acid or salt thereof or polyvinylpyrollidone.

3.    A composition according to claim 2 wherein the binder is an optionally substituted $C_{1-6}$ alkyl ether derviative of cellulose.

4.    A composition according to claim 3 wherein the binder is ethyl cellulose or hydroxypropyl methyl cellulose.

5.    A composition according to any one of the preceding claims wherein the salt of mupirocin is an alkali metal salt.

6.    A composition according to claim 5 wherein the alkali metal salt is the calcium salt, in particular the crystalline dihydrate.

7.    A composition according to any one of the preceding claims which further comprises a diluent.

8.    A composition according to claim 9 wherein the diluent is microcrystalline cellulose, lactose, sucrose, dicalcium phosphate, starch, soya meal, calcium carbonate or whey powder.

0183424

9.    A composition according to claim 7 or claim 8
wherein the diluent is microcrystalline cellulose or
lactose.

10.   A process for preparing a composition according to
claim 1 which process comprises admixing mupirocin or a
veterinarily acceptable salt thereof in a solvent and
removing the solvent.

11.   A method of promoting growth in animals which
method comprising administering to the animal an
effective amount of a composition according to claim 1.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X,Y | GB-A-1 577 545 (BEECHAM GROUP)<br><br>* Claims 1,6,8,9; page 2, lines 8-24; page 2, line 64 - page 3, line 9 *<br><br>--- | 1,2,5,<br>7-9,11 | A 61 K 47/00<br>A 61 K 31/35<br>A 23 K 1/17<br>A 23 K 1/00<br>A 61 K 9/00 |
| Y | EP-A-0 093 808 (BEECHAM GROUP)<br><br>* Claims 1-3,6,10; page 2, line 24 - page 3, line 3 *<br><br>--- | 1,5,7,<br>8,11 | |
| Y | US-A-3 627 885 (J. RONDELET et al.)<br><br>* Claims 1,5,6; column 1, lines 50-75; column 2, lines 9-30; column 6, example 8 * | 1-4,7,<br>8,10,<br>11 | |
| A | | 1 | **TECHNICAL FIELDS SEARCHED (Int Cl.4)** |
| A | ---<br>US-A-4 048 268 (N.H. LUDWIG)<br>* Claim 1 * | 1,2 | A 61 K<br>A 23 K |
| A | ---<br>GB-A-1 458 431 (MEIJI SEIKA K.K.)<br>* Claims 1-3,6,7,11 *<br><br>----- | 1-4 | |

The present search report has been drawn up for all claims

| Place of search | Date of completion of the search | Examiner |
|---|---|---|
| THE HAGUE | 20-02-1986 | DEKEIREL M.J. |